# EUROPÄISCHE PATENTSCHRIFT

(11) **EP 1 553 987 B1**
(45) Veröffentlichungstag und Bekanntmachung des Hinweises auf die Patenterteilung: **01.08.2007**
(21) Anmeldenummer: 03785635.8
(22) Anmeldetag: 24.10.2003
(51) Int. Cl.: A61K 51/12

(54) **VERFAHREN ZUR BESTIMMUNG DER MAGENENTLEERUNG MIT EINER 13C-MARKIERTEN TESTMAHLZE**
METHOD FOR DETERMINING GASTRIC EVACUATION USING A 13C-LABELLED TEST MEAL
METHODE D'ETUDE DE LA VIDANGE GASTRIQUE AU MOYEN D'UN REPAS D'EPREUVE MARQUE AU 13C

(30) Priorität: 26.10.2002 DE 10249929
(43) Veröffentlichungstag der Anmeldung: 20.07.2005
(73) Patentinhaber: Infai Institut für Biomedizinische Analytik und NMR-Imaging GMBH, 44799 Bochum (DE)
(72) Erfinder: AYGEN, Sitke, INFAI Institut, 51105 Köln (DE)
(74) Vertreter: Schreiber, Christoph
(86) Internationale Anmeldenummer: PCT/EP2003/011787
(87) Internationale Veröffentlichungsnummer: WO 2004/037298

(56) Entgegenhaltungen:
- SCHADEWALDT P ET AL: "APPLICATION OF OSOTOPE-SELECTIVE NONDISPERSIVE INFRARED SPECTROMETRY (IRIS) FOR EVALUATION OF 13COCTANOIC ACID GASTRIC-EMPTYING BREATH TESTS: COMPARISON WITH ISOTOPE RATIO-MASS SPECTROMETRY (IRMS)" CLINICAL CHEMISTRY, AMERICAN ASSOCIATION FOR CLINICAL CHEMISTRY. WINSTON, US, Bd. 43, Nr. 3, 1997, Seiten 518-522, XP001018908 ISSN: 0009-9147
- JOON SEONG LEE ET AL: "TOWARD OFFICE-BASED MEASUREMENT OF GASTRIC EMPTYING IN SYMPTOMATIC DIABETICS USING Ä13CÜOCTANOIC ACID BREATH TEST" AMERICAN JOURNAL OF GASTROENTEROLOGY, NEW YORK, NY, US, Bd. 95, Nr. 10, Oktober 2000 (2000-10), Seiten 2751-2761, XP008013990 ISSN: 0002-9270
- SYMONDS E L ET AL: "Assessment of gastric emptying in the mouse using the [13C]-octanoic acid breath test." CLINICAL AND EXPERIMENTAL PHARMACOLOGY & PHYSIOLOGY. AUSTRALIA SEP 2000, Bd. 27, Nr. 9, September 2000 (2000-09), Seiten 671-675, XP002270704 ISSN: 0305-1870
- DUAN L-P ET AL: "INFLUENCE OF CISAPRIDE ON GASTRIC EMPTYING OF SOLIDS AND LIQUIDS MONITORED BY 13C BREATH TESTS" DIGESTIVE DISEASES AND SCIENCES, PLENUM PUBLISHING CO, US, Bd. 40, Nr. 10, Oktober 1995 (1995-10), Seiten 2200-2206, XP001079012 ISSN: 0163-2116
- KATALOG ALDRICH-ISOTEC, [Online] XP002270705 USA Gefunden im Internet: <URL:http://www.sigmaaldrich.com/suite7/Ar ea_of_Interest/Organic___Inorganic_Chemist ry/stable_Isotopes__Isotec_/Applications/B reath_Test.html> [gefunden am 2004-02-18]
- KATALOG CAMBRIDGE ISOTOPE LABS, [Online] XP002270706 USA Gefunden im Internet: <URL:http://www.isotope.com/cil/products/l istproducttypes.cfm?prodtypeid=34> [gefunden am 2004-02-18]
- SCHOONJANS R. ET AL: "The 13C-octanoic acid breath test: validation of a new noninvasive method of measuring gastric emptying in rats" NEUROGASTROENTEROLOGY AND MOTILITY, Bd. 14, Nr. 3, Juni 2002 (2002-06), Seiten 287-293,
- DEBRECENI A. ET AL: "Capsaicin increases gastric emptying rate in healthy human subjects measured by 13C-labeled octanoic acid breath test" JOURNAL OF PHYSIOLOGY, Bd. 93, Nr. 5, November 1999 (1999-11), Seiten 455-460,

## Beschreibung

Gegenstand der vorliegenden Erfindung ist ein Verfahren zur Bestimmung der Magenentleerung durch orale Applikation einer Testsubstanz und Messung der Verweilzeit im Magen.

Der bisherige sogenannte "Goldstandard" dieser Bestimmung besteht darin, dass dem Patienten ein ^{99m}Tc-Eiweiskolloid oral appliziert wird, woraufhin durch Radioscintigraphie festgestellt wird, wo sich dieses radioaktive Isotop befindet.

Dieser Test führt zu einer an sich unerwünschten radioaktiven Belastung der zu untersuchenden Person, so dass bisher auf eine Validierung der Testergebnisse bei der gleichen Person unter gleichen Bedingungen verzichtet wurde. Ein weiterer Nachteil dieser Methode ist, dass die Verweilzeit auch bei Gesunden erheblichen Schwankungen unterworfen ist, die unter anderem auch erklärt werden können durch die unterschiedliche Form des Magens. Weiterhin führen bereits stärkere Bewegungen des Körpers zu einer Veränderung der Position des radioaktiven Markers und dabei zu falschen Ergebnissen.

Untersuchungen der Geschwindigkeit der Magenentleerung mit dem Salz der ¹⁴C-Octansäure und ¹³C-Octansäure mit einer Testmahlzeit hat zu deutlich unterschiedlichen Ergebnissen geführt, die vor allem auch erhebliche Abweichungen aufwiesen, von den Werten, die nach dem "Goldstandard" ermittelt worden sind. Als Testmahlzeit wurde dabei die ¹⁴C-Octansäure und ¹³C-Octansäure als Salz in einem Omelettteig eingerührt und dann gebacken, wobei man davon ausging, dass ein solches Omelett bei gesunden Patienten innerhalb einer relativ kurzen Zeit soweit angedaut ist, dass es im Duodenum absorbiert wird; vgl. Ghoos et al., Gastroenterologie 1993; 104: Seiten 1640 bis 1647.

Ein für den Patienten weniger belastendes Bestimmungsverfahren zur Bestimmung der Magenentleerung verzichtet auf die Verwendung von ¹⁴C- Octansäure, vgl. Schadewaldt et al., Clinical Chemistry 1997; 43: Seiten 518-522, sowie Pfaffenbach et al., Z. Gastroenterol. 1995 ; 33 : Seiten 141 - 145.

Hierzu ist anzumerken, dass bei Verwendung der freien ¹³C-Octansäure in an Eigelb gebundener Form eine optimale Andauung im Magen stattfindet, so dass bei gesunden Personen diese Testmahlzeit in relativ kurzer Zeit in den Duodenum abgeleitet wird, wo dann die ¹³C-Octansäure über das Portalsystem in die Leber transportiert und dort zu ¹³CO₂ oxidiert wird. Das so gebildete ¹³CO₂ wird dann in der ausgeatmeten Atemluft mittels IRMS gemessen. IRMS (Isotopic Ratio Mass Spectrography) ist inzwischen die empfindlichste und beste Methode zur Bestimmung von ¹³CO₂ in der ausgeatmeten Atemluft.

Die optimierte Testmahlzeit besteht z.B aus einem Spiegelei, in welches die freie ¹³C-Octansäure eingerührt wird, danach zusammen mit einer Scheibe Toastbrot, 5 bis 10 g Margarine oder Butter verzehrt wird, woraufhin 150 ml Wasser oder Kaffee getrunken werden. Die Menge an ¹³C-Octansäure beträgt im allgemeinen 40 bis 100 mg. Geeignet ist beispielsweise zu 99% angereicherte ¹³C-Octansäure der Firma Isotec, Miamsbrough, OH, USA.

Erfindungsgemäß hat sich nun gezeigt, dass auf die von Ghoos et al., Loc. Cit. geforderten aufwendigen körperbezogenen Umrechnungsfaktoren wie Bodyindex verzichtet werden kann, da diese bei der Berechnung der Magenentleerung eine wesentlich geringere Rolle spielen als der Schwankungsbereich zwischen der normalen - verzögerten - stark verzögerten Magenentleerung. Beim erfindungemäßen Verfahren wird durch Änderung der Dosierung nur die Höhe der Kurve, nicht aber der zeitliche Verlauf der Metabolisierung und somit der Zeitpunkt der Maximierung beeinflusst. Aus den anliegenden Kurven und Daten vom Verlauf der ¹³CO₂-Ausscheidung bei fünf verschiedenen Patienten zeigt, dass bei normaler Magenentleerung das Maximum der ¹³CO₂-Ausscheidung bei > 2,9 h und die Halbwertzeit bei < 90 min liegt (vgl. Nr. 1). Bei verzögerter Magenentleerung liegt das Maximum bei 2,9 bis 2,5 h und die Halbwertzeit zwischen 90 und 120 min (vgl. Nr. 2 und 3). Bei stark verzögerter Magenentleerung liegt das Maximum bei < 2,5 h und die Halbwertzeit bei > 120 min (vgl. Nr. 4). Bei einer etwas schnelleren Magenentleerung liegt das Maximum bei > 2,9 h und die Halbwertzeit bei < 90 h (vgl. Nr. 5). Deshalb benötigt man drei bis vier Stunden zur Probennahme und zur optimalen Berechnung. Der Abstand der Probennahme ist optimal bei 15 min.

Diese Werte wurden bei den gleichen Patienten verglichen mit den Ergebnissen der einmaligen Szintographie nach Siegel und zeigten nur eine relativ gute Übereinstimmung.

Wiederholung bei gleichen Patienten nach dem erfindungsgemäßen Verfahren zeigte hingegen eine wesentlich bessere Übereinstimmung, was sich aus den obigen Erläuterungen erklärt.

Abweichungen von normalen Zeiten der Magenentleerung können Hinweise liefern auf die verschiedensten Krankheiten wie Gastritis, gastrische Karzinome, Diabetes (Ketoacidose und Gastroparese), Hypothyroidismus, Uremie, Hyperkalieimia, Hypercalcemia, hepatitisches Koma, Magenoperationen wie postoperativer Ileus, Vagotomie oder Resektion des Magens sowie neurologische Störungen des zentralen Nervensystems oder Schädigungen durch anticholinergische oder opoide Arzneimittel, trizyklische Antidepressiva etc. Eine zu schnelle Entleerung ist andererseits der Hinweis auf das Zollinger-Ellison-Syndrom, Vagotomie und Pyloroplastie/Antrectomie, Einfluss von Drogen wie Casaprid, Domperidone, Metoclopramid etc. Ungeeignet ist das erfindungsgemäße Verfahren eigentlich nur bei Erkrankungen von Leber oder Pankreas sowie bei Duodenum-Reflux. Ein Vorteil des erfindungsgemäßen Verfahrens ist hingegen, dass es wegen der unproblematischen Wiederholbarkeit auch zur Verlaufskontrolle von Therapien eingesetzt werden kann.

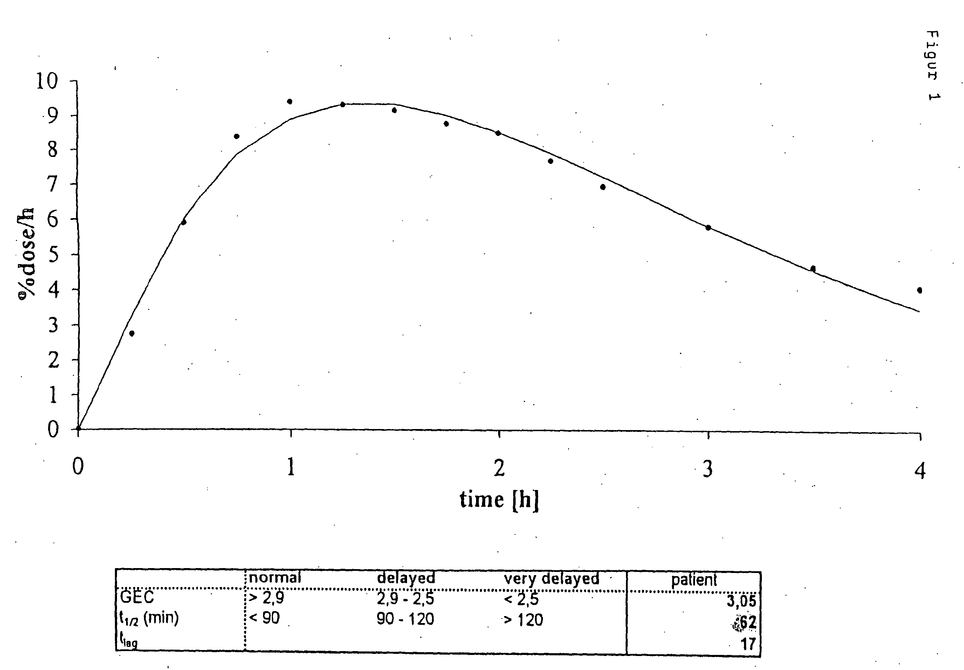

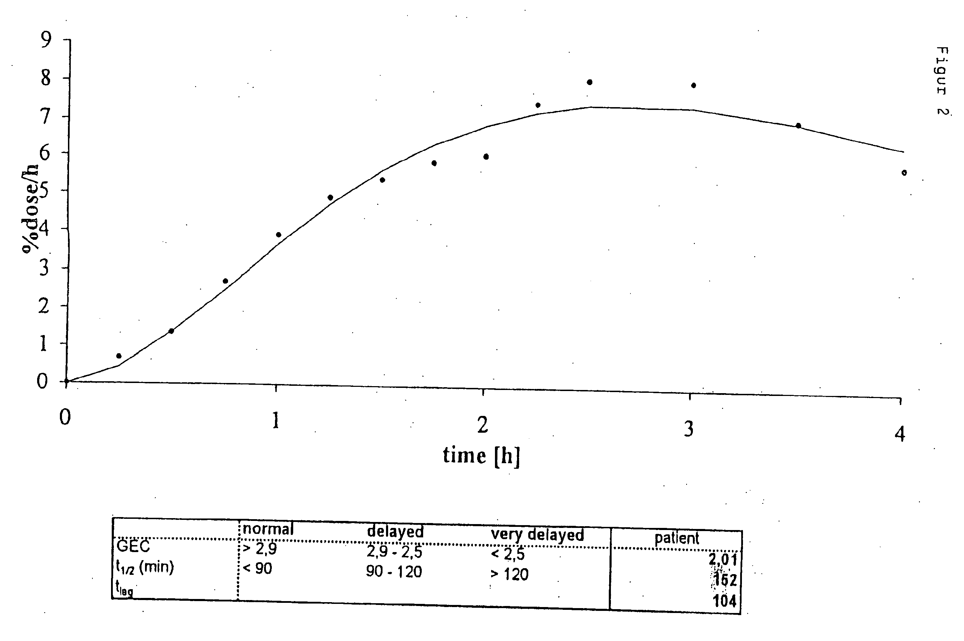

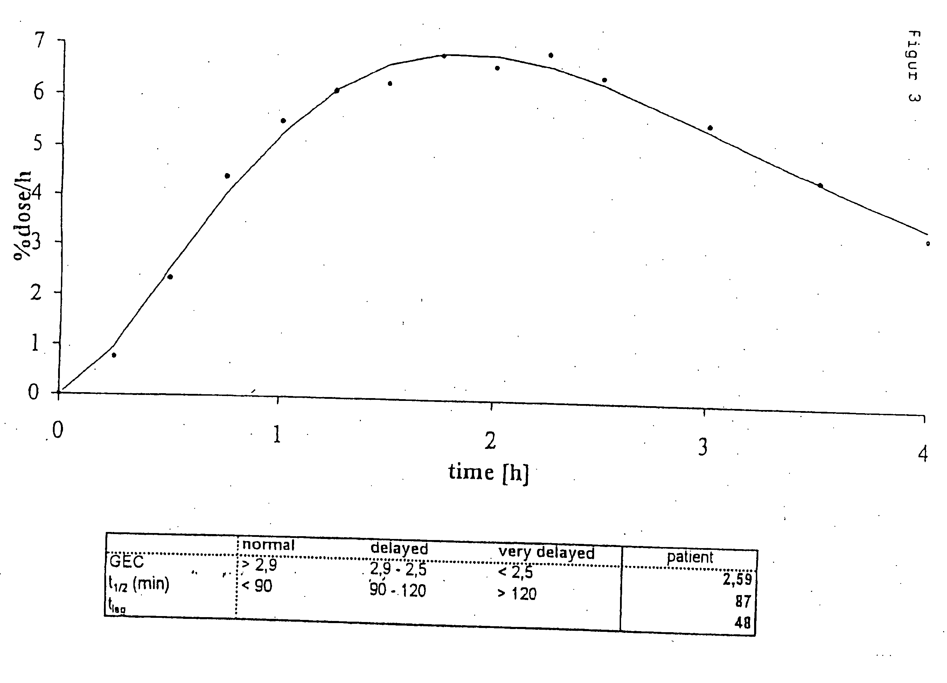

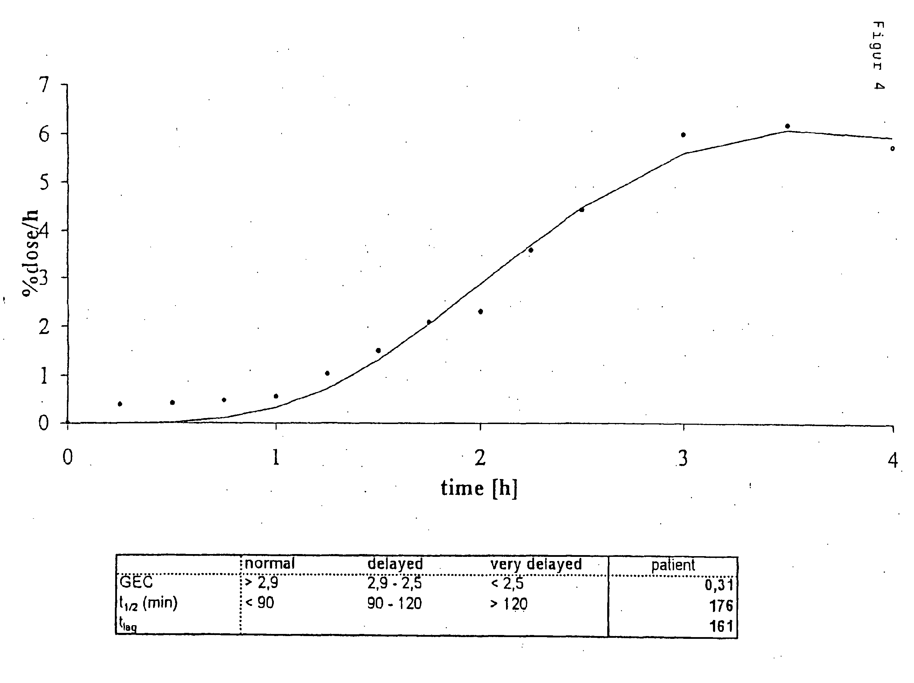

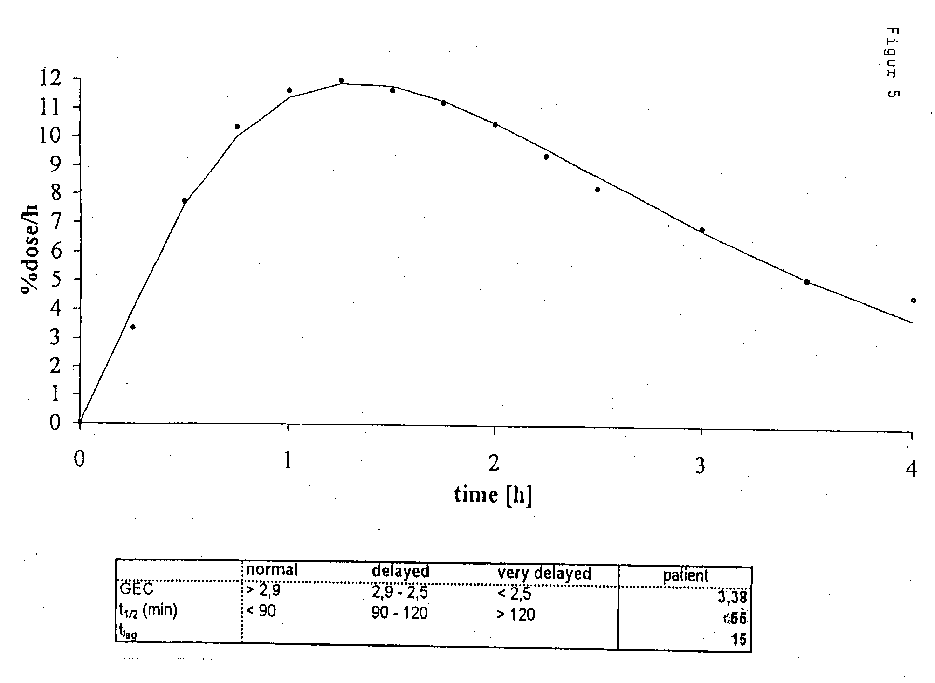

## Patentansprüche

1. Verfahren zur Bestimmung der Magenentleerung eines Menschen durch orale Applikation einer Testsubstanz und Messung der Verweilzeit im Magen, wobei vor und nach der oralen Applikation von freier ¹³C-Octansäure in an Eigelb gebundener Form zusammen mit einer standardisierten Testmahlzeit der Anstieg von ¹³CO₂ in der ausgeatmeten Atemluft mittels IRMS bestimmt wird, **dadurch gekennzeichnet, dass** auf körperbezogene Umrechnungsfaktoren verzichtet wird.

2. Verfahren gemäß Anspruch 1, **dadurch gekennzeichnet, dass** die Testmahlzeit besteht aus einem Spiegelei, in welches die freie ¹³C-Octansäure eingerührt wird, danach zusammen mit einer Scheibe Toastbrot, 5 bis 10 g Margarine oder Butter verzehrt wird, woraufhin 150 ml Wasser oder Kaffee getrunken werden.

## Claims

1. A method for determining the gastric emptying of a human by orally administering a test substance and measuring its dwelling time in the stomach, wherein the increase of ¹³CO₂ in the exhaled respiratory air is determined by means of IRMS before and after the oral administration of free ¹³C-octanoic acid in a form bound to egg yolk together with a standardized test meal, **characterized in that** body-related conversion factors are dispensed with.

2. The method according to claim 1, **characterized in that** said test meal consists of a fried egg into which the free ¹³C-octanoic acid is stirred, whereupon it is eaten together with a slice of toasting bread, 5 to 10 g of margarine or butter, followed by drinking 150 ml of water or coffee.

## Revendications

1. Procédé de détermination de la vidange gastrique chez un être humain par administration orale d'une substance d'essai et mesure du temps de séjour dans l'estomac, dans lequel l'augmentation du ¹³CO₂ dans l'air expiré est déterminée par SMRI avant et après l'administration orale d'acide octanoïque ¹³C libre sous forme incorporée dans du jaune d'oeuf conjointement avec un repas d'épreuve normalisé, **caractérisé en ce que** l'on peut se passer des facteurs de conversion liés à l'organisme.

2. Procédé selon la revendication 1, **caractérisé en ce que** le repas d'épreuve se compose d'un oeuf au plat dans lequel est délayé l'acide octanoïque ¹³C libre, ingéré conjointement avec une tranche de pain grillé, 5 à 10 g de margarine ou de beurre, à la suite de quoi 150 mL d'eau ou de café sont absorbés.
